# EUROPÄISCHE PATENTSCHRIFT

(11) **EP 0 841 987 B1**
(45) Veröffentlichungstag und Bekanntmachung des Hinweises auf die Patenterteilung: **05.01.2000**
(21) Anmeldenummer: 96917490.3
(22) Anmeldetag: 03.06.1996
(51) Int. Cl.: B01J 31/20, C07F 15/06, C07C 45/50

(54) **WASSERLÖSLICHE COBALTKATALYSATOREN, VERFAHREN ZU IHRER HERSTELLUNG UND IHRE VERWENDUNG ALS HYDROFORMYLIERUNGSKATALYSATOREN IN EINEM ZWEIPHASENSYSTEM MIT POLYETHYLENGLYCOL ALS POLARE PHASE**
WATER-SOLUBLE COBALT CATALYSTS, PROCESS FOR THEIR PREPARATION AND THEIR USE AS HYDROFORMYLATION CATALYSTS IN A TWO-PHASE SYSTEM WITH POLYETHYLENE GLYCOL AS THE POLAR PHASE
CATALYSEURS AU COBALT SOLUBLES DANS L'EAU, PROCEDE PERMETTANT DE LES PREPARER ET LEUR UTILISATION COMME CATALYSEURS D'HYDROFORMYLATION DANS UN SYSTEME A DEUX PHASES AVEC DU POLYETHYLENE GLYCOL COMME PHASE POLAIRE

(30) Priorität: 16.06.1995 DE 19521936
(43) Veröffentlichungstag der Anmeldung: 20.05.1998
(73) Patentinhaber: Celanese GmbH, 60439 Frankfurt (DE)
(72) Erfinder: RITTER, Uwe, D-99425 Weimar (DE); WINKHOFER, Norbert, D-37077 Göttingen (DE); ROESKY, Herbert, D-37085 Göttingen (DE)
(86) Internationale Anmeldenummer: EP9602394
(87) Internationale Veröffentlichungsnummer: WO9700132

(56) Entgegenhaltungen:
- US-A- 4 045 493
- US-A- 4 144 191
- J. ORGANOMET. CHEM., Bd. 178, 1979, Seiten 227-247, XP002009593 SEYFERTH , RUDIE, NESTLE: "Organocobalt cluster complexes" in der Anmeldung erwähnt
- JOURN. CHEM. SOC. , CHEM. COMM., Nr. 18, 1980, Seiten 852-853, XP002009594 EVANS, GRACEY: "Generalized cluster anchoring to oxide supports"
- JOURN. AMERICAN SOCIETY, Bd. 99, Nr. 15, 1977, Seiten 5209-5210, XP002009595 SEYFERTH, NIVERT: "organocobalt cluster complexes"

## Beschreibung

Die Erfindung betrifft wasserlösliche Cobaltkatalysatoren, Verfahren zu ihrer Herstellung und ihre Verwendung als Hydroformylierungskatalysatoren in einem Zweiphasensystem mit Polyethylenglycol als polare Phase.

Wasserlösliche Katalysatoren bieten große technische Vorteile wegen der leichten Abtrennbarkeit des Katalysators bei gleichzeitig hoher Selektivität des Katalysators durch die homogene Reaktionsführung. Der durch die leichte Abtrennbarkeit reduzierte Primärenergieaufwand bei durch die hohe Selektivität erreichter gleichzeitiger Verringerung unerwünschter Nebenprodukte führt zu einem steigenden Interesse an Zweiphasenkatalysatoren.

Es bestand daher der Bedarf weitere Verbindungen dieses Typs zur Verfügung zu stellen.

Diese Aufgabe wird gelöst durch Cobaltcarbonylkatalysatoren der Formel (I) 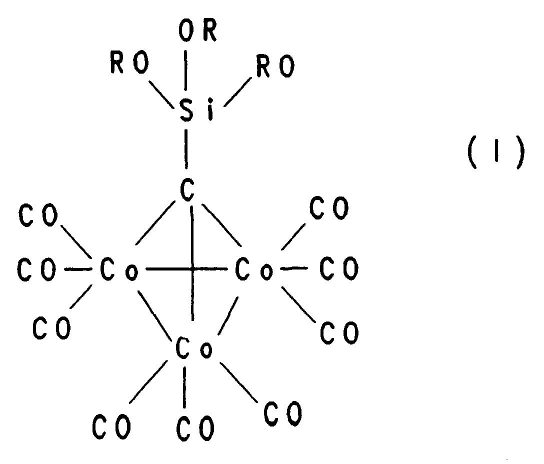 worin R für -CH(CH₂OCH₂CH₃)₂ oder -C₂H₄(OCH₂CH₂)ₙOY steht, wobei n = 1 bis 12 und Y = H oder CH₃ ist.

Die erfindungsgemäßen Verbindungen werden hergestellt, indem Verbindungen der Formel (II) mit Alkoholen ROH umgesetzt werden: 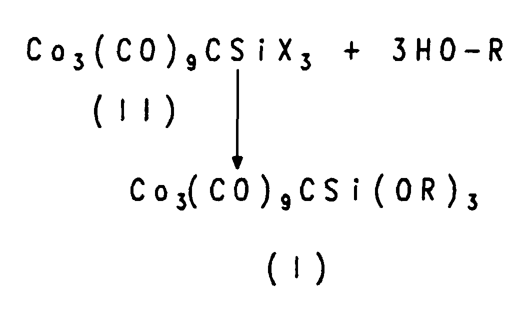
X steht hierbei für OH oder Chlor,
R hat die oben erwähnte Bedeutung
Verbindungen der Formel (II) sind nach D. Seyferth, J. Organomet. Chem. 1979, 178, 227-247 erhältlich.

Verbindungen der Formel (I) zeigen hohe Umsatzraten und Chemoselektivitäten bei der Hydroformylierung von Olefinen. Von Interesse ist insbesondere die Umsetzung von Olefinen der allgemeinen Formel CH₃(CH₂)ₘCH=CH₂ (m = 2 bis 9) in einem Zweiphasensystem mit Polyethylenglycol als polare Phase. Hierbei hat es sich bewährt bei Temperaturen von 50 bis 150°C, Drücken von 50 bis 100 kPa und einem Konzentrationsverhältnis Katalysator:Substrat von 1:20 bis 1:1000 zu arbeiten.

### Beispiel 1

0,574 g (1 mmol) Tricobaltnonacarbonylmethylidintrichlorsilan werden in 10 ml Polyethylen 400 aufgenommen und 24 h lang unter CO-Atmosphäre auf 80°C erwärmt. Anschließend wird vom Bodensatz filtriert. Der CO-Gehalt beträgt 13,75 g/l (ermittelt durch Atomabsorptionsspektroskopie). Die Verbindung ist in Polyethylenglycol 400 gelöst und zeigt eine intensive violette Färbung. Sie ist vollständig mit Wasser mischbar und ist nahezu nicht mischbar mit unpolaren Solventien (Hexan, Pentan u.a.).
Das IR-Spektrum der Lösung zeigt im Bereich der Carbonyle folgende Banden:
1887 cm⁻¹, 1979 cm⁻¹, 1995 cm⁻¹, 2029 cm⁻¹, 2060 cm⁻¹.

### Beispiel 2

0,574 g (1 mmol) Tricobaltnonacarbonylmethylidintrichlorsilan werden in 10 ml 1,3-Diethoxy-2-propanol aufgenommen und 24 h lang unter CO-Atmosphäre auf 60°C erwärmt. Anschließend wird im Hochvakuum der überschüssige Alkohol abdestilliert. Die Verbindung fällt in Form einer violetten hochviskosen Lösung an. Das IR-Spektrum der Verbindung in Nujol zeigt im Bereich der Carbonyle folgende Banden:
2037 cm⁻¹, 2056 cm⁻¹. Das ¹H-NMR-Spektrum zeigt drei Signalgruppen bei:
1,03 ppm (CH₃,d,6H); 3,26 ppm (CH₂,q,4H); 3,42 ppm (CH₂,m,4H); 4,01 ppm (CH,m,1H) (gemessen in d6-Benzen). Das ²⁹Si-NMR-Spektrum zeigt ein Singulett bei -53,8 ppm (gemessen in d8-THF).

### Beispiele 3 bis 6

Verwendung der unter 1 hergestellten Verbindung zur Hydroformylierung von 1-Hexen in Polyethylenglycol 400:
Die Katalysetests werden in einem 100 ml Laborautoklaven durchgeführt. Bei einer typischen Reaktion werden unter Stickstoff 2 ml der Polyethylenglycollösung, die durch Reaktion von Tricobaltnonacarbonylmethylindintrichlorsilan mit Polyethylenglycol 400 in der beschriebenen Weise hergestellt wurde (I), vorgelegt und mit 2 ml 1-Hexen (16 mmol) versetzt. Der Autoklav wird dann mit 70 kPa eines Gemisches (1/1) von CO und H₂ befüllt. Der Ansatz wird unter Rühren 18 h lang auf 120°C erwärmt. Nach der Reaktion werden zur besseren Phasenseparation 4 ml Pentan zugegeben und die Phasen getrennt. Die obere unpolare Phase enthält Pentan und die Reaktionsprodukte, die durch Vergleich mit authentischen Proben gaschromatographisch oder durch GC-MS bestimmt werden. Als interner Standard dient Heptan. Die untere Polyethylenglycolphase enthält den Katalysator und kann ohne Vorbehandlung wiederverwendet werden.
Die Ergebnisse sind in Tabelle 1 zusammengefaßt.

### Beispiele 7 bis 13

Katalysetests mit Co₃(CO)₉CSiX₃ (X = OH) (III) wurden als Vergleichsversuche analog zu den Beispielen 5 bis 8 durchgeführt, wobei die in Tabelle 1 angegebenen Bedingungen eingehalten wurden.

**Tabelle 1**

| Bsp. ^{a} | Temp. °C | Druck Kpa ^{b} | mg Co ^{c} | Ausbeute % ^{d} | Zeit h | Selektivität n/iso ^{e} |
|---|---|---|---|---|---|---|
| 3 | 120 | 70 | 13.75 | 96.5 | 18 | 0.73 |
| 4 | 120 | 70 | 20.6 | 99.5 | 18 | 0.75 |
| 5 | 120 | 70 | 34 | 99.9 | 18 | 0.75 |
| 6^{h} | 120 | 70 | 34 | 72.9 | 18 | 0.67 |
| 7 | 120 | 70 | 10.9 | 96.8 | 24 | 1.4 |
| 8 | 120 | 70 | 7.8 | 98.0 | 24 | 0.79 |
| 9 | 120 | 126 | 7.8 | 97.0 | 12 | 3.75 |
| 10 | 120 | 42 | 7.8 | 25.0 | 72 | 1.81 |
| 11 | 120 | 70 | 7.8 | 98.5 | 72 | 1.93 |
| 12 ^{f} | 120 | 70 | 11.6 | 98.9 | 24 | 3.2 |
| 13 ^{g} | 120 | 70 | 10.2 | 99.8 | 24 | 2.96 |
| CO₂(CO)₈ | 120 | 70 | 22 | 95.6 ⁱ | 18 | 0.58 |

| | | | | | | |
|---|---|---|---|---|---|---|
| ^{a)} Beispiele 3 bis 6 ohne Lösungsmittel, Beispiele 7 bis 13 mit Toluen als Lösungsmittel | | | | | | |
| ^{b)} Startdruck | | | | | | |
| ^{c)} Substrat: 2 ml 1-Hexen | | | | | | |
| ^{d)} Ausbeuten beziehen sich auf die verbrauchte Menge 1-Hexen. Als Hauptprodukte wurden 1-Heptanal, 2-Methylhexanal und 2-Ethylpentanal nachgewiesen. Eine Hydrierung von 1-Hexen und den Aldehyden zu Alkoholen war unter den angegebenen Reaktionsbedingungen nicht zu beobachten. | | | | | | |
| ^{e)} n/iso: 1 Heptanal/2-Methylhexanal + 2 Ethylpentanal | | | | | | |
| ^{f)} Zusatz Triphenylphosphan (Molverhältnis zum Kat. 1:1) | | | | | | |
| ^{g)} Zusatz Bis-diphenylphosphanomethan (Molverhältnis zum Kat. 1:1) | | | | | | |
| ^{h)} wiederverwendeter Katalysator nach Phasenseparation | | | | | | |
| ⁱ⁾ Chemoselektivität: 44 % Aldehyde / 56 % Alkohole | | | | | | |

## Patentansprüche

1. Cobaltcarbonylkatalysator der Formel (I) 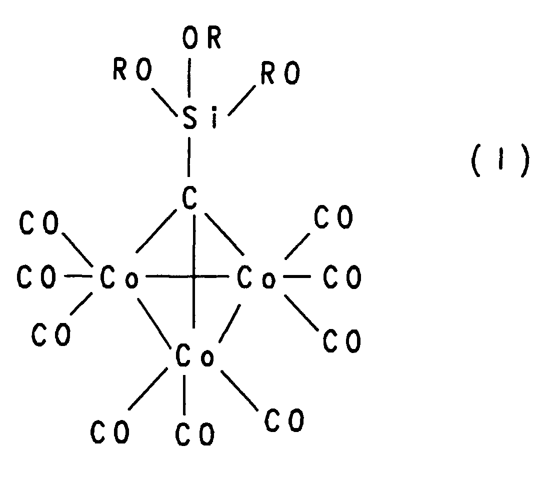 worin R für -CH(CH₂OCH₂CH₃)₂ oder -C₂H₄(OCH₂CH₂)ₙOY steht, wobei n = 1 bis 12 und Y = H oder CH₃ ist.

2. Verfahren zur Herstellung von Verbindung (I), dadurch gekennzeichnet, daß man Verbindung (II)
Co₃(CO)₉CSiX₃ (II)
mit Alkoholen ROH, wobei R die oben aufgeführte Bedeutung hat, und X für OH, Cl steht, umsetzt.

3. Verwendung der Katalysatoren nach Anspruch 1 als Hydroformylierungskatalysatoren.

4. Verwendung nach Anspruch 3, dadurch gekennzeichnet, daß die Hydroformylierung bei Temperaturen von 50 bis 150°C durchgeführt wird.

5. Verwendung nach Anspruch 3 oder 4, dadurch gekennzeichnet, daß die Hydroformylierung bei Drücken von 50 bis 100kPa durchgeführt wird.

6. Verwendung nach mindestens einem der Ansprüche 3 bis 5, dadurch gekennzeichnet, daß das Verhältnis Katalysator:Substrat 1:20 bis 1:1000 beträgt.

7. Verwendung nach mindestens einem der Ansprüche 3 bis 6, dadurch gekennzeichnet, daß als Substrat Olefine der allgemeinen Formel
CH₃(CH₂)ₘCH = CH₂
eingesetzt werden, wobei m = 2 bis 9 ist.

## Claims

1. Cobalt carbonyl catalyst of the formula (I) 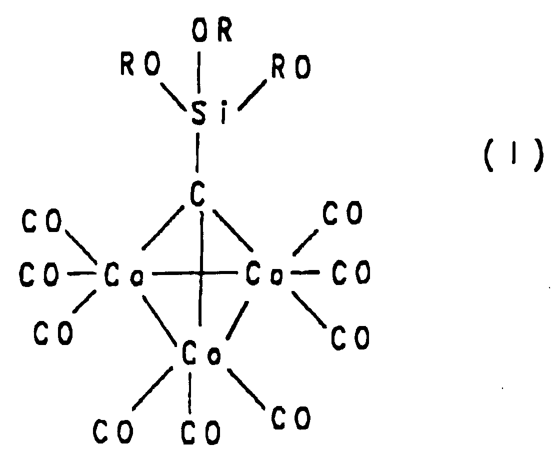 where R stands for -CH(CH₂OCH₂CH₃)₂ or -C₂H₄(OCH₂CH₂)ₙOY in which n = 1 to 12 and Y = H or CH₃.

2. A process for preparing compound (I), characterised in that compound (II)
Co₃(CO)₉CsiX₃ (II)
is reacted with alcohols ROH where R has the aforementioned meaning and X stands for OH, Cl.

3. The use of the catalysts according to claim 1 as hydroformylation catalysts.

4. The use according to claim 3, characterised in that the hydroformylation is performed at temperatures of 50 to 150°C.

5. The use according to claim 3 or 4, characterised in that the hydroformylation is conducted at pressures of 50 to 100 kPa.

6. The use according to at least one of claims 3 to 5, characterised in that the ratio of catalyst to substrate is 1:20 to 1:1000.

7. The use according to at least one of the claims 3 to 6, characterised in that olefins of the formula
CH₃(CH₂)ₘCH = CH₂
are used as the substrate, where m = 2 to 9.

## Revendications

1. Catalyseur de cobaltcarbonyle de la formule (I) 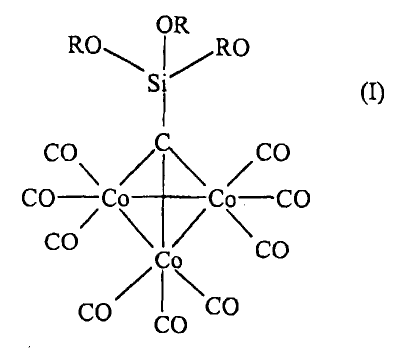 dans laquelle R représente -CH(CH₂OCH₂CH₃)₂ ou -C₂H₄(OCH₂CH₂)ₙOY, n=1 à 12 et Y=H ou CH₃.

2. Procédé pour la préparation de composé (I), caractérisé en ce que l'on fait réagir un composé (II)
Co₃(CO)₉CSiX₃ (II)
avec des alcools ROH, R ayant la signification indiquée ci-dessus et X représentant OH, Cl.

3. Utilisation des catalyseurs selon la revendication 1 comme catalyseurs d'hydroformylation.

4. Utilisation selon la revendication 3, caractérisée en ce que l'hydroformylation est réalisée à des températures de 50 à 150°C.

5. Utilisation selon la revendication 3 ou 4, caractérisée en ce que l'hydroformylation est réalisée à des pressions de 50 à 100 kPa.

6. Utilisation selon au moins l'une quelconque des revendications 3 à 5, caractérisée en ce que le rapport catalyseur:substrat est compris entre 1:20 et 1:1000.

7. Utilisation selon au moins l'une quelconque des revendications 3 à 6, caractérisée en ce que l'on utilise comme substrat des oléfines de la formule générale
CH₃(CH₂)ₘCH=CH₂
m = 2 à 9.
